# EUROPEAN PATENT APPLICATION

(11) **EP 3 730 111 A1**
(43) Date of publication of application: **28.10.2020**
(21) Application number: 19201170.8
(22) Date of filing: 02.10.2019
(51) Int. Cl.: A61F 13/511, A61F 13/49, D04H 3/013

(54) **LIQUID PERMEABLE TOPSHEET AND ABSORBENT HYGIENE ARTICLE CONTAINING SAID TOPSHEET**

(71) Applicant: LENZING AKTIENGESELLSCHAFT, 4860 Lenzing (AT)
(72) Inventor: CARLYLE, Tom, Holly Springs, NC 27540 (US); GOLDHALM, Gisela, 3363 Neufurth (AT); MAYER, Katharina, 4813 Altmünster (AT)

(57) **Abstract**

The invention relates to a liquid permeable topsheet for use in an absorbent hygiene article, comprising at least one nonwoven layer. It is in object of the invention to provide a biodegradable topsheet with improved dimensional stability and liquid wicking properties. To solve the stated problem, it is therefore proposed, that the at least one nonwoven layer is a cellulosic nonwoven layer, directly manufactured from lyocell spinning solution, forming a network of substantially endless regenerated cellulosic fibers.

## Description

### Technical Field

The present invention relates to a liquid permeable topsheet for use in an absorbent hygiene article, comprising at least one nonwoven layer, and an adsorbent hygiene article containing said topsheet.

### Background Art

Topsheets are regularly used in a variety of adsorbent hygiene articles, such as baby diapers, feminine hygiene sheets, adult incontinence diapers, pads and underpads, etc. Topsheets for these products share a number of common requirements for a reliable functionality. They require good liquid wicking properties and high dimensional stability, even in the wet state, as well as high strength and low linting properties. Furthermore fast liquid take-up/ acquisition and good spread-ability of liquids (in order to distribute the liquid uniformly over the surface of the product rather than only near the liquid introduction point) are key parameters. Additionally, biodegradability and sustainability become an ever more important criterion for disposable hygiene articles.

Current nonwoven topsheets, however, fail to fulfill all of the above mentioned needs. Meltspun polyolefins and products incorporating them, on the one hand, are not very absorbent or biodegradable or sustainably sourced. Cellulosic nonwovens or products incorporating cellulosic fibers, on the other hand, typically do not show low linting, or dimensional stability, especially when wet, and may not be strong enough or soft enough for some hygiene applications.

There has been extensive research in the production of nonwoven sheets/layers. For example, US 4,340,563, US 3,849,241 and US 4,863,785 disclose production methods of spunbond, meltblown and spundbond/meltblown/spunbond composite (SMS) nonwoven webs from polyolefins, respectively. Such spunbond or meltblown nonwoven webs show excellent dimensional stability, low linting and high strength, but are not hydrophilic, biodegradable or sustainable, thus, limiting their suitability for topsheet applications. There have been many attempts to correct these deficiencies. The lack of hydrophilicity is addressed by many prior art technologies. US 5,647,862 discloses a method of applying a coating of a surfactant to impart temporary hydrophilicity. However, it also admits that the hydrophilicity is temporary and depletes with each successive liquid insult. US 6,118,218 discloses corona and plasma treatment to impart hydrophilicity to spunlaid polyolefins, but this treatment is transient, diminishing with time in storage. US 5,830,604 discloses a method for chemically grafting hydrophilic polymer chains onto the surface of polyolefins, however this method suffers from high complexity and low cost-efficiency. Finally, US 7,923,597 discloses a method for polymerizing a sheath of hydrophilic polymers over the core of the polyolefin, using a UV curable material. Again, such a method is very complex and costly. Biodegradability and sustainability are also addressed by prior art. US 7,989,062 and US 8,518,311 both disclose a spunlaid bicomponent fiber nonwoven, where the bicomponent fiber is based on two biodegradable polyesters. Again, this is an expensive and complex technology.

Carded and hydroentangled or spunlace nonwovens have been used in topsheet applications. US 5,626,571 teaches the use of both carded and hydroentangled nonwovens for topsheet applications. Hydroentangled or spunlace nonwovens and carded nonwovens are often compromise products, containing both synthetic and cellulosic components. These have marginally liquid wicking and dimensional stability and are usually not biodegradable or sustainable.

Thus, there is distinct need for a strong, soft, absorbent, liquid wicking, fast liquid take up, good liquid spreading, comfortable, dimensionally stable (even wet), low linting, cost effective, biodegradable and sustainable nonwoven material for use in topsheet applications.

### Disclosure of the Invention

Thus it is an object of the present invention to provide a topsheet of the aforementioned type, which shows improved dimensional stability and liquid wicking properties, while at the same time being biodegradable.

The present invention achieves the stated object in that the at least one nonwoven layer is a cellulosic nonwoven layer, directly manufactured from lyocell spinning solution, forming a network of substantially endless regenerated cellulosic fibers.

If the nonwoven fabric is a cellulosic nonwoven fabric, consisting of substantially endless regenerated cellulosic fibers, which form a network of substantially endless regenerated cellulosic fibers, the nonwoven layer being directly manufactured from lyocell spinning solution, an easy to produce yet reliable topsheet with high dimensional stability may be provided. The cellulosic fibers can further improve the liquid wicking and liquid strike through properties of the topsheet.

Furthermore, the inventive topsheet may contain essentially only cellulose, thus, showing good biodegradability. Such an essentially pure cellulose nonwoven layer may be formed directly from lyocell spinning solution and is preferably multibonded by merged filaments, hydrogen bonding and/or physical intermingling of the filaments to form the network of essentially continuous regenerated cellulosic fibers. Thus, the dimensional stability of the topsheet may be further improved.

The cellulosic nonwoven layer, directly manufactured from lyocell spinning solution, constituting the topsheet of the present invention is manufactured according to a novel variant of a spunlaid nonwoven process, e.g. described in WO 2018/184038 and WO 2018/184932. In this process, a (lyocell) cellulose spinning mass is extruded simultaneously through a spinneret containing closely-spaced meltblown jet nozzles. The extruded spinning solution is then attenuated using high velocity air streams and the nonwoven web/layer is formed on a moving surface. Finally the nonwoven web is washed and dried. By carefully adjusting the spinning parameters (cellulose concentration of the spinning mass, spinneret extrusion rate, attenuating air stream velocity, etc.), the strength- and liquid-transport-properties of the formed nonwoven can be tailored to the needs.

Such inventive topsheet formed by the above mentioned process may thus provide fast wicking, fast liquid uptake, good spread-ability of liquid, enhanced dimensional stability, softness, comfort, biodegradability and sustainability; all without the need of adding binders or chemicals.

It is mentioned in general, that the topsheet containing essentially only cellulose takes into account, that regenerated molded bodies, like fibers or filaments spun according to the lyocell process, may contain residual amounts of other substances than cellulose, e.g. hemicelluloses. This does, however, not affect the quality of the product or its ability for use according to this invention in any way.

The at least one nonwoven layer of the topsheet may be additionally treated by hydroentangling, hydroembossing and/or perforating to further improve the dimensional stability of the topsheet.

Further advantageous embodiments of the invention are shown in accordance with the dependent claims 2 to 10.

The present invention further relates to an absorbent hygiene article, comprising an absorbent core and a liquid permeable topsheet according to any of claims 1 to 10 arranged over the surface of the absorbent core.

### Modes for Carrying Out the Invention

In the following, the invention is described in further detail in accordance to a number of preferred embodiments. All described embodiments may be combined with each other without restrictions, if not stated otherwise.

In a first embodiment, the inventive liquid permeable topsheet comprises a first nonwoven layer that is directly manufactured from lyocell spinning solution. The first nonwoven layer contains substantially endless regenerated cellulosic fibers, which form a network of substantially endless regenerated cellulosic fibers. A topsheet with fast liquid uptake, good spread-ability of liquid, good liquid wicking, low linting, good dimensional stability (wet and dry), which is soft and strong and non-irritating can be made. All of this is achieved with the topsheet being 100% biodegradable, compostable and made from 100% sustainable raw materials.

Additionally, the regenerated cellulosic fibers in the first nonwoven layer are multibonded by merging, hydrogen bonding and/or physically intermingling in order to form the network of essentially endless regenerated cellulosic fibers, resulting in a topsheet with high dimensional stability. This multibonding may result directly from the manufacturing process or through additional steps. The advantages of the multibonding used in the inventive topsheet is that high strength, low elongation and good stiffness are all achieved. This combination enables the material to exhibit very good dimensional stability and a high strength as an essentially pure cellulosic material. It also provides the low linting character that comes from good filament tie-down (merged filaments) and does not negatively influence the excellent softness that cellulosic materials are known to exhibit, and this translates into a comfortable, non-irritating material that can be used next to the user's skin.

In another embodiment, the topsheet of the nonwoven material according to the invention is additionally bonded or treated by a hydroentanglement, hydroembossing, perforation, needlepunch or chemical bonding process to modify the physical properties. Such modified properties may be for example bulk density, tensile strength and elongation as well as appearance and haptics.

In a further embodiment, the inventive topsheet has a dry elongation of less than or equal to 40 % in MD (machine direction) and less than or equal to 50 % in CD (cross direction). In a further preferred embodiment, the inventive topsheet has a dry elongation of less than or equal to 30 % in MD, preferably of less than or equal to 20 % in MD, more preferably of less than or equal to 10 % in MD. In another preferred embodiment, the inventive topsheet has a dry elongation of less than or equal to 40 % in CD, preferably of less than or equal to 30 % in CD.

In a further embodiment, the inventive topsheet has a wet elongation of less than or equal to 35 % in MD and less than or equal to 50 % in CD. In a further preferred embodiment, the inventive topsheet has a wet elongation of less than or equal to 25 % in MD, preferably of less than or equal to 15 % in MD. In another preferred embodiment, the inventive topsheet has a wet elongation of less than or equal to 40 % in CD, preferably of less than or equal to 30 % in CD.

The methods for measuring the dry and wet elongation are described in the examples section. All elongation values in this disclosure - if not stated otherwise explicitly - refer to measurements taken in line with this methods.

In another embodiment, the inventive topsheet has a strike through time of less than or equal to 2.6 s. In yet further embodiments, the strike through time is less than or equal to 2.4 s, less than or equal to 2.2 s, or less than or equal to 2.0 s.

The method for measuring the liquid strike through time used throughout this disclosure is described in detail in the examples section.

In a preferred embodiment, the basis weight of the topsheet is between 10 and 30 g/m² (gsm - grams per square meter). Within this range, we find that inventive topsheet product performances can be advantageously achieved.

In a further preferred embodiment, the topsheet comprises multiple cellulosic nonwoven layers of substantially endless regenerated cellulosic fibers which are directly formed on top of each other in one single process step and which are interconnected by means of merging and/or hydroentanglement to form the network of substantially endless regenerated cellulosic fibers. All layers are directly manufactured from lyocell spinning solution on top of each in one process step.

In a further embodiment, the nonwoven layers in the topsheet can be combined with other nonwoven materials through hydroentanglement, hydroembossing and/or perforating. Thereby, the topsheet comprises an additional layer formed on top of the network of substantially endless fibers, whereby the additional layer consists of cellulosic pulp or staple fibers, more particularly lyocell fibers, and the additional layer is hydroentangled, hydroembossed or perforated with the network of substantially endless fibers.

In order to modify wicking, or other liquid handling properties of the nonwoven material for use in topsheet products according to the invention it can be treated with chemicals, polymers or other materials.

In another embodiment, the nonwoven layers within the topsheet are treated with chemicals, polymers or other materials to modify rewet, hydrophobicity or other liquid handling properties. The individual nonwoven layers or the network of substantially endless cellulosic fibers as a whole can be treated accordingly.

The invention also discloses an absorbent hygiene article comprising an absorbent core and a liquid permeable topsheet according to any number of embodiments above, arranged over the surface of the absorbent core.

In another embodiment, the absorbent hygiene article further comprises a backsheet. The absorbent core is then arranged between the topsheet and said backsheet. The liquid permeable topsheet should thereby acquire and pass the liquid quickly to the absorbent core, while the liquid impermeable backsheet should hold the liquid inside the absorbent core.

In further embodiments, such an absorbent hygiene article is a baby diaper, a feminine care pad, an adult incontinence product or a tampon. Nevertheless, many other absorbent hygiene articles, where the properties of the inventive topsheet are beneficial, are in the scope of this invention.

### Examples

In the following, the invention will be illustrated by examples. These examples are not limiting the scope of the invention in any way. The invention also includes any other embodiments which are based on the same inventive concept.

### Example 1:

A 15-gsm product of the invention was tested for liquid spread-ability and liquid strike through time versus other commercial topsheet products of about the same basis weight being comprised of surfactant treated spunbond/meltblown/spunbond (SMS) polypropylene. It is important for a topsheet to have both good liquid spread-ability and fast liquid strike-through.

The results are shown in Table 1. The topsheets according to the invention (inventive examples E1 and E2) had equivalent spread-ability to the commercially available topsheet-products (comparative examples C1 to C8), but drastically improved strike through times of up to 7x faster.

**Table 1: Liquid strike through times measured for 2 inventive examples E1 and E2 and for 8 comparative examples C1 to C8.**

| sample | strike through time [s] |
|---|---|
| E1 (inventive example 1) | 1,6 |
| E1 (inventive example 2) | 2,4 |
| C1 (comparative example 1) | 11,3 |
| C2 (comparative example 2) | 5,7 |
| C3 (comparative example 3) | 3,2 |
| C4 (comparative example 4) | 3,5 |
| C5 (comparative example 5) | 3,2 |
| C6 (comparative example 6) | 3,3 |
| C7 (comparative example 7) | 2,7 |
| C8 (comparative example 8) | 2,8 |

Prior to all measurements, the samples were first conditioned at 23 °C (± 2 °C) and 50 % (± 5 %) relative humidity for 24 h.

The test method for spread-ability was as follows: 0.5 ml of test liquid (water with 2 g/L dye sulfacide brilliant green) was pipetted onto each sample using an Eppendorf pipette. After 1 day, a picture of the liquid spread was taken and software (ImageJ1.49v, National Institute of Health, USA) used to evaluate the area of the liquid spread.

Liquid strike through test is done according to NWSP 070.3.R0(15) using simulated urine. The NWSP 70.3 Liquid Strike-Through test method measures the strike-through time, i.e. the time taken for a known volume of liquid (simulated urine) applied to the surface of a test portion of nonwoven coverstock, which is in contact with an underlying standard absorbent pad, to pass through the nonwoven.

### Example 2:

Furthermore, the topsheets according to the invention (inventive examples E1 and E2) and the commercially available topsheets (comparative examples C1 to C3) were tested for dimensional stability, in particular elongation in machine direction (MD) and cross direction (CD). Machine direction thereby refers to the direction parallel to the direction of movement of the moving surface the nonwoven web is formed upon during production. Cross direction refers to the direction perpendicular to the machine direction.

The results are depicted in Table 2. The inventive examples E1 and E2 show a significantly improved dimensional stability with respect to the comparative examples C1, C2 and C3.

**Table 2: Dry and wet elongation in MD and CD measured for 2 different inventive examples E1 and E2 and 3 comparative examples C1, C2 and C3.**

| sample | dry elongation MD [%] | dry elongation CD [%] | wet elongation MD [%] | wet elongation CD [%] |
|---|---|---|---|---|
| E1 (inventive example 1) | 4,1 | 10,8 | 10,2 | 21,7 |
| E1 (inventive example 2) | 3,1 | 25,1 | 12,3 | 25,8 |
| C1 (comparative example 1) | 48,1 | 58,7 | 48,3 | 56,8 |
| C2 (comparative example 2) | 47,4 | 72,3 | 39,7 | 66,0 |
| C3 (comparative example 3) | 43,1 | 62,5 | 41,4 | 63,5 |

Prior to all measurements, the samples were first conditioned at 23 °C (± 2 °C) and 50 % (± 5 %) relative humidity for 24 h. For all measurements, the samples had a size of 5 cm x 10 cm (width x length). The clamping length was 8 cm and the speed for testing 10 cm/min.

Elongations in MD and CD (in dry and wet state) were measured according to DIN EN 29 073 part 3 / ISO 9073-3 (in the version of year 1992).

To determine dry elongation, the samples were measured after the conditioning described above.

To determine wet elongation, the samples were wetted out 3 fold with demineralized water and were sealed within a plastic bag for 60 min to equilibrate moisture.

## Claims

1. A liquid permeable topsheet for use in an absorbent hygiene article, comprising at least one nonwoven layer, **characterized in that**, the at least one nonwoven layer is a cellulosic nonwoven layer, directly manufactured from lyocell spinning solution, forming a network of substantially endless regenerated cellulosic fibers.

2. The topsheet according to claim 1, **characterized in that** the topsheet has a dry elongation of less than or equal to 40 % in MD, more particularly of less than or equal to 30 % in MD, preferably of less than or equal to 20 % in MD, more preferably of less than or equal to 10 % in MD, and of less than or equal to 50 % in CD, more particularly of less than or equal to 40 % in CD, preferably of less than or equal to 30 % in CD.

3. The topsheet according to claim 2, **characterized in that** the topsheet has a wet elongation of less than or equal to 35 % in MD, more particularly of less than or equal to 25 % in MD, preferably of less than or equal to 15 % in MD, and of less than or equal to 50 % in CD, more particularly of less than or equal to 40 % in CD, preferably of less than or equal to 30 % in CD.

4. The topsheet according to any of claims 1 to 3, **characterized in that** the topsheet has a strike through time of less than or equal to 2.6 s, more particularly of less than or equal to 2.4 s, preferably of less than or equal to 2.2 s.

5. The topsheet according to any of claims 1 to 4, **characterized in that** the at least one cellulosic nonwoven layer exhibits modified physical properties due any or any combination of additional hydroentanglement, hydroembossing, perforating, needlepunching and chemical bonding processes.

6. The topsheet according to any of claims 1 to 5, **characterized in that** the substantially endless regenerated cellulosic fibers in the cellulosic nonwoven layer are multibonded by merging, hydrogen bonding and/or physical intermingling of said fibers.

7. The topsheet according to any of claims 1 to 6, **characterized in that** the topsheet comprises multiple cellulosic nonwoven layers of substantially endless regenerated cellulosic fibers which are directly formed on top of each other in one single process step and which are interconnected by means of merging and/or hydroentanglement to form the network of substantially endless regenerated cellulosic fibers.

8. The topsheet according to any of claims 1 to 7, **characterized in that** the topsheet comprises an additional layer formed on top of the network of substantially endless fibers, whereby the additional layer consists of cellulosic pulp or staple fibers, more particularly lyocell fibers, and the additional layer is hydroentangled, hydroembossed or perforated with the network of substantially endless fibers.

9. The topsheet according to any of claims 1 to 8, **characterized in that** the topsheet has a basis weight between 10 and 30 g/m².

10. The topsheet according to any of claims 1 to 9, **characterized in that** the topsheet has a modified absorbency, wicking, or other liquid handling property due to a treatment with chemicals or polymers.

11. An absorbent hygiene article comprising an absorbent core and a liquid permeable topsheet according to any of claims 1 to 10 arranged over the surface of the absorbent core.

12. The absorbent hygiene article according to claim 11, **characterized in that** it further comprises a backsheet, whereby the absorbent core is arranged between the topsheet and said backsheet.

13. The absorbent hygiene article according to any of claims 11 or 12, **characterized in that** the absorbent hygiene article is a baby diaper, feminine care pad, adult incontinence pad or tampon.
